# FASCICULE DE BREVET EUROPEEN

(11) **EP 3 877 741 B1**
(45) Date de publication et mention de la délivrance du brevet: **10.05.2023**
(21) Numéro de dépôt: 19872255.5
(22) Date de dépôt: 06.11.2019
(51) Int. Cl.: G01N 1/08, A61B 10/00, G01N 1/38

(54) **DISPOSITIF DE PRÉPARATION D'AU MOINS UN ÉCHANTILLON PAR CAROTTAGE D'UN PRÉLÈVEMENT DE MATIÈRE**
VORRICHTUNG ZUR VORBEREITUNG VON MINDESTENS EINER PROBE DURCH KERNBOHREN EINER MATERIALPROBE
DEVICE FOR PREPARING AT LEAST ONE SAMPLE BY CORING A SAMPLE OF MATERIAL

(30) Priorité: 09.11.2018 FR 1860388
(43) Date de publication de la demande: 15.09.2021
(73) Titulaire: Commissariat à l'Énergie Atomique et aux Énergies Alternatives, 75015 Paris (FR); Elimaje, 86000 Poitiers (FR)
(72) Inventeur: REVOL-CAVALIER, Frédéric, 38054 Grenoble cedex (FR); ALESSIO, Manuel, 38054 Grenoble cedex (FR); CUBIZOLLES, Myriam-Laure, 38054 Grenoble cedex (FR); LAURENT, Patricia, 38054 Grenoble cedex (FR); NIEF, Nadège, 38054 Grenoble cedex (FR)
(74) Mandataire: INNOV-GROUP
(86) Numéro de dépôt international: PCT/FR2019/052634
(87) Numéro de publication internationale: WO 2020/094983

(56) Documents cités:
- WO-A1-2009/136445
- WO-A1-2017/153863
- FR-A1- 3 023 614
- US-A1- 2008 227 662

## Description

### Domaine technique de l'invention

La présente invention se rapporte à un dispositif de préparation d'au moins un échantillon à partir d'un prélèvement de matière.

Le dispositif est notamment adapté pour réaliser, en vue d'analyses ultérieures, de manière rapide et fiable un ou plusieurs échantillons calibrés à partir d'un même prélèvement de matière, celui-ci étant suffisamment mou pour être découpé.

Le dispositif sera notamment adapté pour préparer un ou plusieurs échantillons à partir d'un prélèvement de selle en vue de la détection d'hémoglobine humaine, pour le dépistage du cancer colorectal.

Le dispositif pourra également être adapté à la préparation d'un ou plusieurs échantillons à partir d'un même prélèvement effectué dans :
- La boue pour la recherche de bactéries ou de champignons,
- Le sol pour la recherche de polluants, ou de composés particuliers (sulfates, fer, cuivre...),
- Des végétaux (par exemple champignons, salades, légumes, graines, fruits...) pour effectuer un suivi suite à une contamination (présence de pesticides, bactéries...),
- Des animaux, par exemple dans le cas d'une biopsie d'oreille sur du bétail.

### Etat de la technique

Dans le domaine de la prévention du cancer colorectal, plusieurs systèmes de collecte d'échantillons existent déjà. Ils se basent sur un prélèvement des selles réalisé à l'aide d'un outil de type écouvillon, bandelette, spatule... Ces outils sont maniés directement par l'utilisateur du test qui doit faire ses propres prélèvements et ils permettent de réaliser un prélèvement unitaire qui est calibré par la forme de l'outil.

Une solution connue et disponible consiste à utiliser une canule pour réaliser un prélèvement de selles. La canule est ensuite insérée dans un flacon muni d'un système de septum pour limiter l'excès de matière et ainsi mieux calibrer l'échantillon.

Une autre solution connue consiste à employer un pot dans lequel les selles sont récupérées puis à réaliser un prélèvement par grattage à l'aide d'une canule.

Dans le domaine des prélèvements de matière dans le sol, il existe également des systèmes permettant de réaliser un carottage calibré à l'aide d'un échantillonneur de terrain. Ces dispositifs connus sont constitués d'une tige creuse qui se replie lorsqu'on l'enfonce dans le sol.

Dans le domaine de la lutte contre la diarrhée virale bovine (BVD), un prélèvement est effectué sur le bétail sous la forme d'une biopsie auriculaire. Après contention de l'animal, une pince spécifique est utilisée afin de récupérer un échantillon de cartilage directement sur l'oreille du bovin, grâce à l'utilisation d'une boucle à biopsie. Cette boucle ou trocart est ensuite placée dans un tube de transport qui est scellé avec la même pince afin de permettre son expédition vers le laboratoire d'analyses.

De manière générale, les différentes solutions de préparation d'un échantillon issu d'un prélèvement de selles, de boue, de matière du sol, ou d'origine animale dans le cas de la réalisation d'une biopsie, présentent les inconvénients suivants :
- Manipulation peu pratique,
- Calibration de l'échantillon peu fiable,
- Contamination possible de l'échantillon,
- Inadaptation au domaine d'application (notamment au domaine médical pour les dispositifs de type échantillonneur de terrain),
- Echantillonnage souvent unitaire.

Ces inconvénients, qui peuvent apparaître dans la préparation de l'échantillon, auront ensuite des conséquences sur la fiabilité et sur la pertinence des résultats des analyses effectuées ultérieurement.

Des solutions de prélèvement de matière sont notamment décrites dans les demandes de brevets US 2008/227662 A1, WO2017/153863A1 et FR3023614A1**.**

Le but de l'invention est de proposer un dispositif de préparation d'un ou plusieurs échantillons solides calibrés à partir d'un même prélèvement de matière telle que l'une des matières citées ci-dessus, ledit dispositif étant d'une manipulation facile, sans risque de contamination du prélèvement, ni des échantillons préparés, permettant une calibration aisée de chaque échantillon préparé. Le dispositif permet ainsi de garantir un certain niveau de pertinence des résultats lors d'une analyse ultérieure de chaque échantillon.

### Exposé de l'invention

Ce but est atteint par un dispositif de préparation d'un ou plusieurs échantillons solides calibrés pour analyse à partir d'un même prélèvement de matière, le dispositif comportant un corps et une structure à trois étages superposés, ladite structure comprenant :
- Un premier étage comprenant une ou plusieurs cuvettes solidaires chacune du corps et destinées chacune à recevoir un liquide de dissolution ou de resuspension et présentant chacune une ouverture,
- Un deuxième étage comprenant un support solidaire du corps qui comporte, au moins un opercule perforable recouvrant l'ouverture de chaque cuvette, ledit opercule présentant une surface de dépôt du prélèvement,
- Un troisième étage qui comporte un dispositif d'échantillonnage comprenant un ou plusieurs emporte-pièces mobiles par rapport au corps,
- Chaque emporte-pièce coopérant en translation avec ledit corps suivant une direction principale, entre au moins un état de repos dans lequel l'emporte-pièce est à l'aplomb de la surface de dépôt et un état actionné dans lequel l'emporte-pièce est situé à l'intérieur de ladite cuvette.
- Le corps comportant un ensemble formé d'un réceptacle et d'un couvercle fermant ledit réceptacle, ledit ensemble délimitant un espace interne, dans lequel est placé le support comportant la surface de dépôt, et un espace externe
- Des moyens mécaniques d'actionnement en translation desdits emporte-pièces.

Selon une particularité, le premier étage est amovible par rapport aux deux autres étages du dispositif.

Selon une autre particularité, le dispositif comporte un canal de liaison connecté à chaque cuvette.

Selon une première réalisation particulière, chaque emporte-pièce est solidaire du couvercle.

Selon une particularité, les moyens mécaniques d'actionnement comportent un mécanisme de glissière agencé entre ledit couvercle et ledit réceptacle pour guider en translation ledit couvercle par rapport au réceptacle suivant ladite direction principale.

Selon une deuxième réalisation particulière, les moyens mécaniques d'actionnement comportent un ensemble mobile dont est solidaire chaque emporte-pièce, situé dans l'espace interne et actionnable en translation à l'aide d'un organe de préhension, un mécanisme de glissière étant agencé entre ledit ensemble mobile et le corps du dispositif.

Selon une particularité, l'opercule est un film plastique ou métallique.

Selon une autre particularité, le dispositif comporte une grille agencée dans ledit réceptacle, en périphérie de la surface de dépôt.

Selon une autre particularité, chaque emporte-pièce est réalisé par une partie creuse à une extrémité libre d'un tube cylindrique de révolution creux dont l'axe est orienté suivant la direction principale.

Selon une autre particularité, le dispositif comporte au moins deux emporte-pièces de volumes distincts.

Selon une autre particularité, le dispositif comporte des cuvettes supplémentaires destinées chacune à recevoir un liquide de dissolution ou de resuspension, non associées à un emporte-pièce du dispositif.

L'invention concerne également l'utilisation du dispositif tel que défini ci-dessus, pour réaliser à partir d'un même prélèvement de selles humaines un ou plusieurs échantillons à l'aide de chaque emporte-pièce.

L'invention concerne un procédé de préparation de plusieurs échantillons calibrés à partir d'un même prélèvement de matière, ledit procédé étant mis en oeuvre à l'aide du dispositif tel que défini ci-dessus et comportant les étapes suivantes :
- Mise en place du prélèvement sur la surface de dépôt alors que le dispositif est à l'état repos,
- Actionnement du dispositif pour entraîner en translation chaque emporte-pièce en direction de la surface de dépôt, entre l'état repos et l'état actionné,
- Découpe par chaque emporte-pièce d'une partie du prélèvement pour créer un échantillon,
- Perforation par chaque emporte-pièce de l'opercule,
- Insertion de chaque emporte-pièce dans une cuvette distincte,
- Dissolution ou re-suspension de l'échantillon dans le liquide présent dans la cuvette.

### Brève description des figures

D'autres caractéristiques et avantages vont apparaître dans la description détaillée qui suit faite en regard des figures annexées listées ci-dessous :
- Les figures 1A à 1C illustrent, de manière schématique, le principe de fonctionnement d'un dispositif de préparation d'un ou plusieurs échantillons de matière, conforme à l'invention et réalisé selon un premier mode de réalisation.
- Les figures 2A à 2C illustrent, de manière schématique, le principe de fonctionnement d'un dispositif de préparation d'un ou plusieurs échantillons de matière, conforme à l'invention et réalisé selon un deuxième mode de réalisation.
- Les figures 3A et 3B illustrent, de manière schématique, le principe de fonctionnement d'un dispositif de préparation d'un ou plusieurs échantillons de matière, conforme à l'invention et réalisé selon un troisième mode de réalisation.
- Les figures 4A et 4B représentent deux variantes de réalisation d'un emporte-pièce employé dans le dispositif de l'invention, réalisé selon l'un des trois modes de réalisation.
- La figure 5 illustre un exemple de réalisation d'une cuvette telle qu'employée dans le dispositif de l'invention.

### Description détaillée d'au moins un mode de réalisation

L'invention vise un dispositif de préparation d'un ou plusieurs échantillons de matière à partir d'un même prélèvement P.

De manière avantageuse, on verra que le dispositif de l'invention permet de préparer plusieurs échantillons de matière de manière simultanée à partir du même prélèvement P de matière (avantageusement molle). De manière non limitative, le dispositif pourra permettre la préparation de un à dix échantillons à partir d'un même prélèvement. Sur les figures annexées, la représentation schématique du dispositif montre la préparation simultanée de deux échantillons distincts à partir d'un même prélèvement P de matière.

Comme déjà décrit ci-dessus, les applications du dispositif sont nombreuses. Pour résumer, il s'applique notamment à la préparation d'échantillons, à partir d'un prélèvement P d'une matière suffisamment molle pour être découpée.

A titre d'exemple, la matière pourra être des selles d'un être vivant (notamment humain), de la boue, de la matière présente dans le sol. Mais le prélèvement P pourra également être de type végétal (des graines de tailles différentes) ou d'origine animal.

Dans le cas de selles d'origine humaine, les échantillons générés pourront notamment servir à détecter la présence d'hémoglobine pour dépister le cancer colorectal.

De manière non limitative, on peut considérer que la matière du prélèvement P qui sert de base à la préparation des échantillons doit être suffisamment molle pour être carottée par la seule force de l'utilisateur. Pour les selles, la dureté du prélèvement pourra être comprise entre 1 et 7 sur l'échelle de Bristol. Cependant, pour certains prélèvements plus durs (comme par exemple des grains durs), il est possible d'utiliser le dispositif uniquement pour calibrer un échantillon (sans découpage/carottage du prélèvement), notamment dans le cas où la taille du prélèvement s'avère plus réduite que la taille de l'emporte-pièce qui est employé dans le dispositif.

Le dispositif de l'invention peut se présenter sous la forme d'un ensemble monobloc, facilement transportable.

Pour la suite de la description et sur les figures annexées, on définit un axe principal (A) suivant une direction verticale, perpendiculaire à la surface sur laquelle est posé ledit dispositif de l'invention. Cette surface peut notamment être le sol ou tout autre support plan.

Les termes "supérieur", "inférieur", "dessus", "dessous" ou équivalents utilisés ci-dessous dans la description sont à comprendre en se référant audit axe principal (A).

Le dispositif de l'invention comporte un corps définissant un espace interne V formé par l'assemblage d'un réceptacle 10, 100 et d'un couvercle 11, 110 venant se positionner sur le réceptacle pour le refermer. Le réceptacle est avantageusement destiné à être posé sur le sol ou sur tout support approprié. Lorsque le couvercle 11, 110 est apposé sur le réceptacle, l'espace interne V est avantageusement isolé de l'extérieur de manière hermétique.

De manière non limitative, comme représenté sur les figures annexées, le réceptacle 10, 100 peut être de forme cylindrique, présentant un fond, une ou plusieurs parois latérales et une ouverture supérieure destinée à être obturée par le couvercle. Bien entendu, toute autre forme pourrait être adaptée.

Le dispositif peut comporter un support réalisé sous la forme d'une plateforme logé dans l'espace interne V du réceptacle. La plateforme comporte une surface de dépôt S du prélèvement à partir duquel est préparé un ou plusieurs des échantillons. Cette surface de dépôt S est avantageusement plane et orientée perpendiculairement à l'axe principal (X).

La plateforme comporte un opercule 16, 160 perforable dont la surface supérieure constitue la surface de dépôt S du prélèvement. De manière non limitative, l'opercule peut être réalisé sous la forme d'un film plastique, polymère, élastomère (par exemple une mousse) ou métallique venant recouvrir de manière hermétique une ou plusieurs cuvettes 13, 130 situées sous la plateforme. La perforation est rendue possible par un simple appui contre la surface de l'opercule suivant une direction transversale à ladite surface S, préférentiellement normale à ladite surface. Cette direction de perforation est donc avantageusement parallèle à l'axe principal (A) défini ci-dessus. On verra que l'appui permettant la perforation de l'opercule 16, 160 est mis en oeuvre à l'aide d'au moins un emporte-pièce 12, 120 du dispositif.

Chaque cuvette 13, 130 est destinée à recevoir un liquide 14, 140 choisi pour permettre la dissolution ou la re-suspension de l'échantillon préparé. Une cuvette 13, 130 distincte est avantageusement dédiée à un échantillon particulier préparé à partir du prélèvement. Le nombre de cuvettes n'est pas limitatif. Il pourra être compris entre un et dix, comme le nombre d'échantillons à préparer ou même être supérieur au nombre d'échantillons à préparer. Dans ce dernier cas, les cuvettes supplémentaires pourront servir d'étalon ou de contrôle, par exemple pour conserver une référence et élaborer un suivi du vieillissement dans le temps des solutions embarquées dans les cuvettes.

Le dispositif de préparation de l'invention comporte un dispositif d'échantillonnage destiné à créer un ou plusieurs échantillons à partir du prélèvement P de matière déposé sur la surface de dépôt S de la plateforme.

Pour chaque échantillon à préparer, le dispositif d'échantillonnage comporte un emporte-pièce 12, 120 distinct destiné à découper le prélèvement P de matière pour réaliser un échantillon calibré.

De manière avantageuse, le dispositif d'échantillonnage comporte plusieurs emporte-pièces 12, 120 en parallèle, permettant de préparer plusieurs échantillons de manière simultanée à partir d'un même prélèvement P.

Le dispositif d'échantillonnage est agencé de manière à permettre une translation des emporte-pièces 12, 120 suivant une direction donnée, normale par rapport à la surface de dépôt S et donc parallèle à l'axe principal (X).

Chaque emporte-pièce 12, 120 peut être réalisé sous la forme d'une partie creuse à l'extrémité libre d'un tube, la partie creuse du tube définissant le volume de la carotte créée lors de la préparation de l'échantillon et donc le volume de l'emporte-pièce. Les tubes sont disposés parallèles entre eux et sont arrangés de sorte que leur axe est parallèle à l'axe principal (A). Chaque tube présente une extrémité fixée à une partie mobile du dispositif (couvercle ou ensemble mobile) et son extrémité libre qui comporte la partie creuse formant l'emporte-pièce. Bien entendu, toute autre forme pourrait être adaptée, à partir du moment où l'emporte-pièce permet de réaliser un carottage du prélèvement.

Selon un aspect particulier de l'invention, on peut envisager différentes configurations du dispositif, notamment des moyens mécaniques d'actionnement distincts des emporte-pièces.

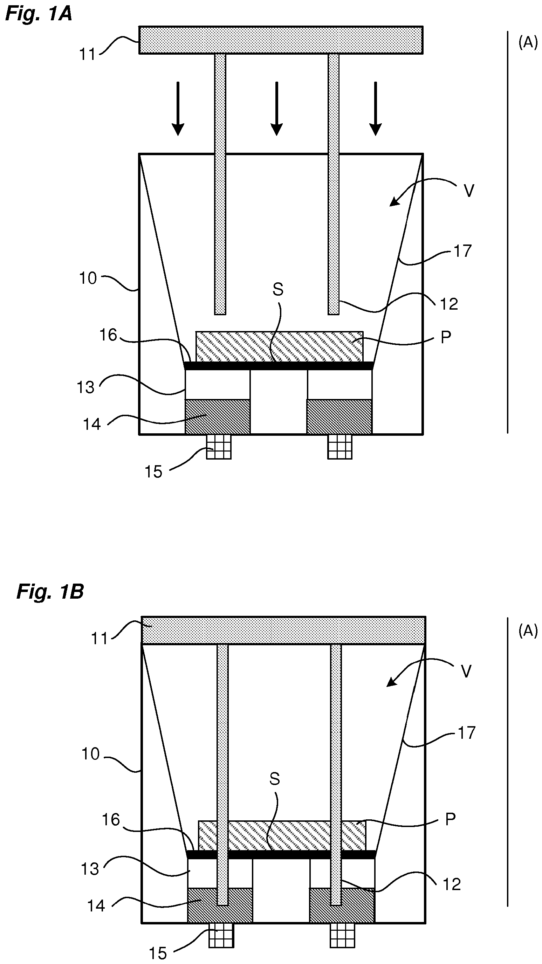

Dans une première configuration représentée sur les figures 1A à 1C, les emporte-pièces 12 sont directement portés par le couvercle 11 du dispositif. Ils sont alors orientés vers l'intérieur du réceptacle 10 lorsque le couvercle 11 est apposé sur le réceptacle 10 pour le refermer. Un mécanisme de glissière peut être prévu entre le réceptacle 10 et le couvercle 11 pour guider le positionnement du couvercle 11 sur le réceptacle 10 suivant la direction de l'axe principal (A). En venant apposer le couvercle sur le réceptacle suivant le mouvement de translation, chaque emporte-pièce 12 vient découper une partie du prélèvement.

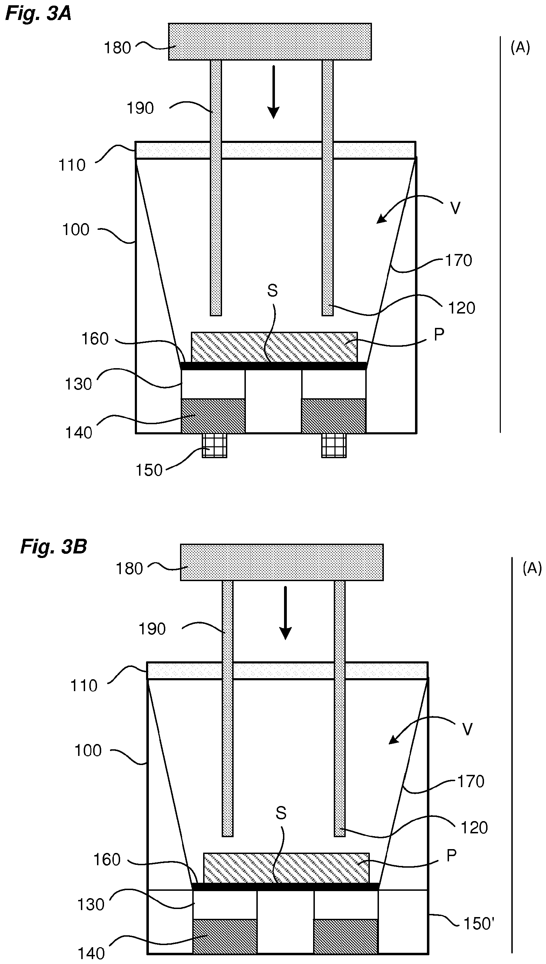

Dans une deuxième configuration représentée sur les figures 3A et 3B, le dispositif d'échantillonnage peut comporter un mécanisme mobile coopérant en coulissement avec le corps du dispositif. De manière non limitative, ce mécanisme mobile peut comporter un organe de préhension 180 situé à l'extérieur du corps du dispositif, le rendant ainsi accessible pour une prise manuelle. Cet organe de préhension 180 peut se présenter sous la forme d'une poignée. Le mécanisme mobile comporte un ensemble mobile en translation dans l'espace interne V du corps du dispositif. Ledit organe de préhension 180 est relié à cet ensemble mobile par l'intermédiaire d'au moins une tige de liaison 190. Ladite tige de liaison est agencée pour traverser le corps du dispositif, à travers au moins une ouverture, formant ainsi un mécanisme de glissière entre le mécanisme mobile et le corps du dispositif. La tige de liaison 190 permet de transmettre le mouvement de translation effectué sur l'organe de préhension à l'ensemble mobile. La liaison peut être réalisée à travers le couvercle 110 du dispositif, qui est alors fixe et qui fait partie du corps du dispositif. Comme évoqué ci-dessus, l'ensemble mobile comporte alors un ou plusieurs emporte-pièces 120 destinés chacun à découper une partie du prélèvement P disposé sur la surface de dépôt S.

Dans ces deux configurations, on peut distinguer deux états distincts du dispositif :
- Un état de repos dans lequel les emporte-pièces 12, 120 ont leur extrémité libre située à l'aplomb du prélèvement P disposé sur la surface de dépôt S. Autrement dit, dans cet état de repos, l'extrémité libre de chaque emporte-pièce 12, 120 est située au-dessus du prélèvement. Dans la première configuration évoquée ci-dessus, le couvercle 11 n'est donc pas refermé sur le réceptacle. Dans la deuxième configuration, l'ensemble mobile est relevé et l'organe de préhension 180 émerge par rapport au couvercle 110.
- Un état actionné dans lequel l'extrémité libre de chaque emporte-pièce 12, 120 vient se loger dans une cuvette 13, 130 correspondante. Dans la première configuration, le couvercle 11 vient alors refermer le réceptacle. Dans la deuxième configuration, l'ensemble mobile est translaté vers le bas par pression sur l'organe de préhension 180 suivant l'axe principal (A).

Entre ses deux états, chaque emporte-pièce 12, 120 est amené d'abord à traverser le prélèvement P pour le découper puis à perforer l'opercule 16, 160 recouvrant une ou plusieurs cuvettes. Si le prélèvement P ne peut pas être découpé (car trop petit par exemple dans le cas d'une graine), l'emporte-pièce 12, 120 permet de calibrer l'échantillon.

Comme déjà décrit, chaque cuvette 13, 1130 est destinée à être remplie d'un liquide permettant la dissolution ou la re-suspension de l'échantillon.

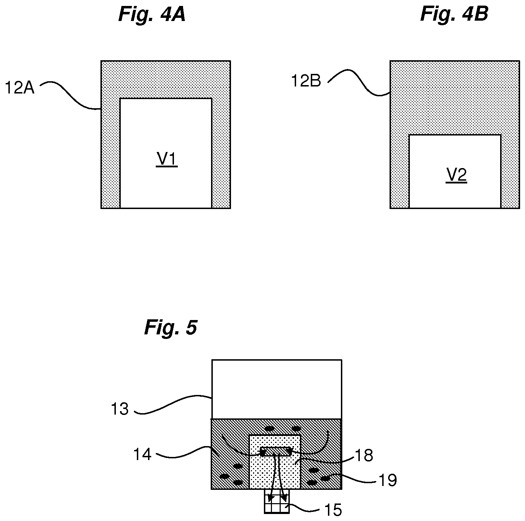

Pour faciliter l'analyse des échantillons préparés et transférés dans le liquide 14, 140 présent dans les cuvettes, deux solutions sont possibles :
Dans une première solution, le dispositif peut comporter un canal de liaison 15, 150 connecté sur chaque cuvette. Le canal 15, 150 permet de transférer le liquide présent dans la cuvette vers un réservoir ou même directement vers un système d'analyse via une connectique fluidique adaptée. Dans cette solution, comme représenté sur la figure 5, il est possible de prévoir une cheminée 18 à l'intérieur de la cuvette, permettant de laisser passer le liquide 14 à récupérer vers le canal de liaison et de retenir dans la cuvette 13 des particules 19 qui pourraient venir fausser l'analyse.

Dans une deuxième solution, la partie du dispositif qui porte les cuvettes 13, 130 peut être amovible. Une fois les échantillons transférés dans les cuvettes, cette partie peut être désolidarisée du reste du réceptacle, scellée pour conservation avant analyse ou envoyée directement pour une analyse par un automate.

Selon une réalisation particulière, chaque emporte-pièce peut comporter des orifices réalisés de manière adaptée pour favoriser l'introduction du liquide de dissolution à travers l'emporte-pièce et accélérer la dissolution de l'échantillon prélevé. Dans le cas d'un emporte-pièce en forme de tube, ces orifices pourront être réalisés sur la paroi latérale et dimensionnés de manière à permettre une diffusion du liquide de dissolution, sans autoriser l'extraction de l'échantillon en dehors de l'emporte-pièce lors de la découpe.

Selon une réalisation particulière, le dispositif peut comporter une grille 17, 170 agencée à l'intérieur du réceptacle 10, 100, autour de la plateforme, réalisée sous la forme d'un ou plusieurs flancs latéraux. Cette grille 17, 170 permet, si nécessaire, de rincer le prélèvement avec une solution de rinçage (ou de détergent), avant la préparation des échantillons. Le liquide de rinçage est dirigé vers le fond du réceptacle. Dans le cas d'un recueil de selles humaines ou animales, la grille 17, 170 permet également de séparer l'urine des selles.

Selon un aspect particulier de l'invention, comme représenté sur les figures 4A et 4B, il est possible d'utiliser des emporte-pièces 12A, 12B de différentes tailles afin de mettre en oeuvre une gamme de détection. Il s'agira par exemple de réaliser des carottes de volumes calibrés V1, V2 différents.

Par exemple, dans le cas où l'ordre de grandeur de la concentration d'une cible d'intérêt à doser est totalement inconnu, l'utilisation d'une gamme de dilution du prélèvement P permettrait de s'assurer que la quantification est toujours faisable. Par exemple, dans le cas d'un immuno-essai (comme l'ELISA), on pourrait aboutir systématiquement à un dosage fiable, que la concentration de l'analyte à doser soit très forte ou très faible dans le prélèvement P de départ.

De manière plus précise, les figures 1A à 1C illustrent le principe de fonctionnement du dispositif selon un premier mode de réalisation. Dans ce premier mode de réalisation, le dispositif présente une configuration dans laquelle le couvercle 11 est mobile en translation par rapport au réceptacle 10. Chaque cuvette 13 est reliée à un canal de liaison 15.

Figure 1A : Le dispositif est à l'état repos et le couvercle 11 est relevé par rapport au réceptacle 10. Le prélèvement P est posé sur la surface de dépôt S. Le prélèvement P peut être rincé en injectant un liquide de rinçage qui sera alors amené à traverser la grille 17.

Figure 1B : Le dispositif est à l'état actionné et le couvercle 11 est refermé sur le réceptacle 10. Chaque emporte-pièce 12 est entraîné en translation lorsque le couvercle 11 est apposé sur le réceptacle 10. En translation, chaque emporte-pièce 12 vient carotter le prélèvement P puis percer l'opercule 16 qui est au-dessous pour pénétrer dans une cuvette 13 distincte. L'échantillon présent dans chaque emporte-pièce 12 est alors dissout par le liquide présent dans sa cuvette 13. Il peut alors être transféré via un canal de liaison 15.

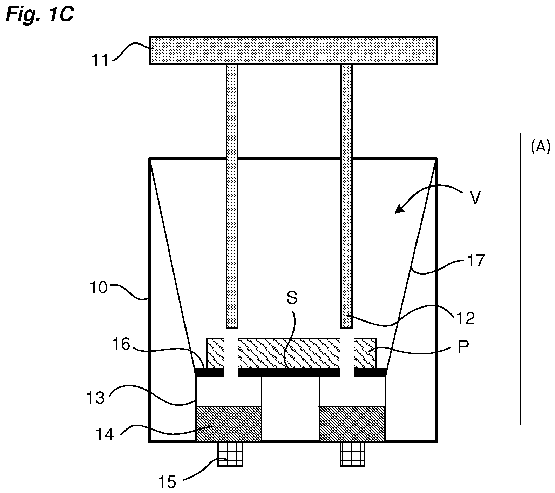

Figure 1C : De manière optionnelle, le dispositif peut ensuite être ramené à l'état repos, après actionnement. Sur cette figure, le prélèvement P est donc diminué des carottes réalisées et l'opercule 16 est percé aux différents points.

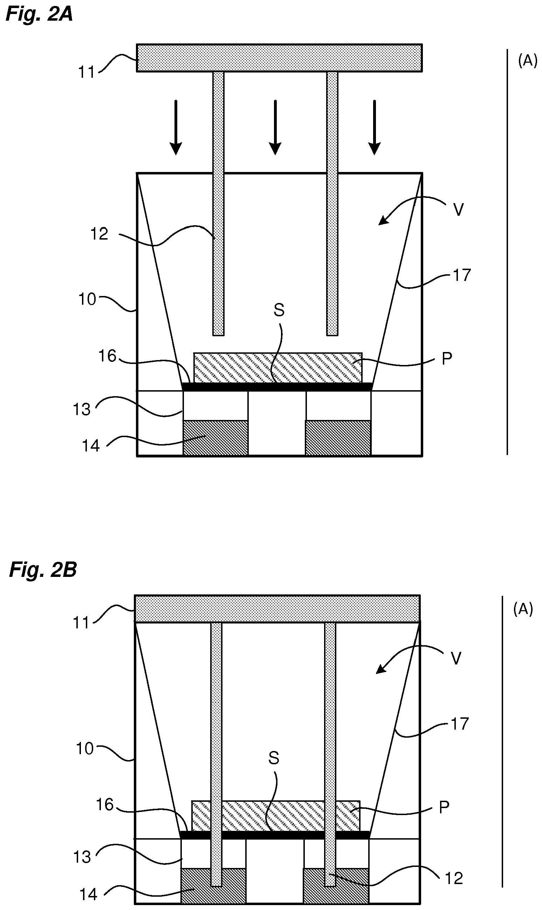

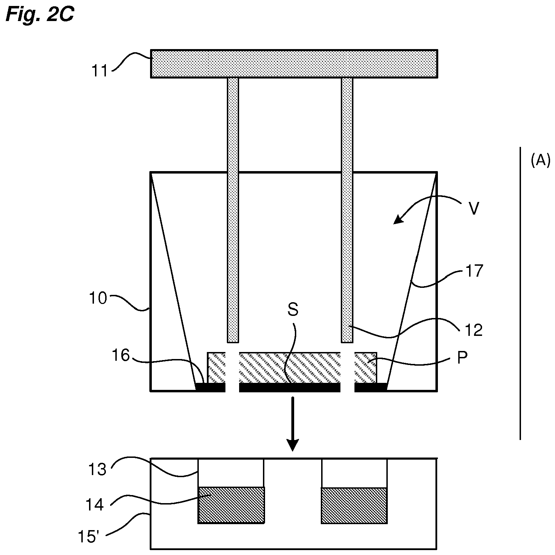

Les figures 2A à 2C sont identiques en fonctionnement à celles des figures 1A à 1C. La différence réside dans le fait que l'étage 15' du dispositif qui inclut les cuvettes est amovible et peut être désolidarisé du reste du dispositif pour être envoyé en analyse. La figure 2C illustre ce principe.

Les figures 3A et 3B représentent la variante de réalisation dans laquelle les emporte-pièces sont entraînés en translation par le mécanisme mobile en actionnant l'organe de préhension 180. Sur la figure 3A, le dispositif est à canal de liaison 15 et sur la figure 3B, le dispositif est à étage 150' amovible pour être envoyé en analyse.

On comprend de ce qui précède que la solution de l'invention présente de nombreux avantages, parmi lesquels :
- Une solution simple pouvant être manipulé directement par l'utilisateur ;
- Une possibilité de créer plusieurs échantillons de manière simultanée à partir d'un même prélèvement de matière ;
- Une solution permettant un transfert direct des échantillons créés vers un système d'analyse, sans risquer une contamination ;

## Revendications

1. Dispositif de préparation d'un ou plusieurs échantillons solides calibrés pour analyse à partir d'un même prélèvement (P) de matière, **caractérisé en ce qu'**il comporte un corps et une structure à trois étages superposés, ladite structure comprenant :
- Un premier étage comprenant une ou plusieurs cuvettes (13, 130) solidaires du corps et destinées chacune à recevoir un liquide (14, 140) de dissolution ou de re-suspension et présentant chacune une ouverture,
- Un deuxième étage comprenant un support solidaire du corps qui comporte, au moins un opercule (16, 160) perforable recouvrant l'ouverture de chaque cuvette, ledit opercule présentant une surface de dépôt (S) du prélèvement,
- Un troisième étage qui comporte un dispositif d'échantillonnage comprenant un ou plusieurs emporte-pièces (12, 120) mobiles par rapport au corps,
- Chaque emporte-pièce (12, 120) coopérant en translation avec ledit corps suivant une direction principale (A), entre au moins un état de repos dans lequel l'emporte-pièce (12, 120) est à l'aplomb de la surface de dépôt (S) et un état actionné dans lequel l'emporte-pièce est situé à l'intérieur de ladite cuvette (13, 130), et **en ce que** :
- Le corps comporte un ensemble formé d'un réceptacle (10, 100) et d'un couvercle (11, 110) fermant ledit réceptacle, ledit ensemble délimitant un espace interne (V), dans lequel est placé le support comportant la surface de dépôt (S), et un espace externe,
- Des moyens mécaniques d'actionnement en translation desdits emporte-pièces.

2. Dispositif selon la revendication 1, **caractérisé en ce que** le premier étage (15', 150') est amovible par rapport aux deux autres étages du dispositif.

3. Dispositif selon la revendication 1, **caractérisé en ce qu'**il comporte un canal de liaison (15, 150) connecté à chaque cuvette (13, 130).

4. Dispositif selon la revendication 1, **caractérisé en ce que** chaque emporte-pièce (12) est solidaire du couvercle (11).

5. Dispositif selon la revendication 4, **caractérisé en ce que** lesdits moyens mécaniques d'actionnement comportent un mécanisme de glissière agencé entre ledit couvercle (11) et ledit réceptacle (10) pour guider en translation ledit couvercle par rapport au réceptacle suivant ladite direction principale (A).

6. Dispositif selon la revendication 1, **caractérisé en ce que** les moyens mécaniques d'actionnement comportent un ensemble mobile dont est solidaire chaque emporte-pièce, situé dans l'espace interne (V) et actionnable en translation à l'aide d'un organe de préhension (180), un mécanisme de glissière étant agencé entre ledit ensemble mobile et le corps du dispositif.

7. Dispositif selon l'une des revendications 1 à 6, **caractérisé en ce que** l'opercule (16, 160) est un film plastique ou métallique.

8. Dispositif selon l'une des revendications 1 à 7, **caractérisé en ce qu'**il comporte une grille (17, 170) agencée dans ledit réceptacle, en périphérie de la surface de dépôt (S).

9. Dispositif selon l'une des revendications 1 à 8, **caractérisé en ce que** chaque emporte-pièce est réalisé par une partie creuse à une extrémité libre d'un tube cylindrique de révolution creux dont l'axe est orienté suivant la direction principale.

10. Dispositif selon la revendication 9, **caractérisé en ce qu'**il comporte au moins deux emporte-pièces de volumes distincts.

11. Dispositif selon l'une des revendications 1 à 10, **caractérisé en ce qu'**il comporte des cuvettes supplémentaires destinées chacune à recevoir un liquide (14, 140) de dissolution ou de re-suspension, non associées à un emporte-pièce du dispositif.

12. Utilisation du dispositif tel que défini dans l'une des revendications 1 à 11, pour réaliser à partir d'un même prélèvement (P) de selles humaines un ou plusieurs échantillons à l'aide de chaque emporte-pièce (12, 120).

13. Procédé de préparation de plusieurs échantillons calibrés à partir d'un même prélèvement (P) de matière, **caractérisé en ce qu'**il est mis en oeuvre à l'aide du dispositif tel que défini dans l'une des revendications 1 à 12, et **en ce qu'**il comporte les étapes suivantes :
- Mise en place du prélèvement (P) sur la surface de dépôt alors que le dispositif est à l'état repos,
- Actionnement du dispositif pour entraîner en translation chaque emporte-pièce (12, 120) en direction de la surface de dépôt, entre l'état repos et l'état actionné,
- Découpe par chaque emporte-pièce d'une partie du prélèvement (P) pour créer un échantillon,
- Perforation par chaque emporte-pièce (12, 120) de l'opercule (16, 160),
- Insertion de chaque emporte-pièce (12, 120) dans une cuvette (13, 130) distincte,
- Dissolution ou re-suspension de l'échantillon dans le liquide (14, 140) présent dans la cuvette (13, 130).

## Patentansprüche

1. Vorrichtung zur Vorbereitung einer oder mehrerer kalibrierter fester Proben für eine Analyse aus ein und derselben entnommenen Materialprobe (P), **dadurch gekennzeichnet, dass** sie einen Körper und eine Struktur mit drei übereinander angeordneten Stufen aufweist, wobei die Struktur umfasst:
- eine erste Stufe, die eine oder mehrere Küvetten (13, 130) umfasst, die mit dem Körper fest verbunden sind und jeweils dazu bestimmt sind, eine Auflösungs- oder Resuspensionsflüssigkeit (14, 140) aufzunehmen, und jeweils eine Öffnung aufweisen,
- eine zweite Stufe, die einen mit dem Körper fest verbundenen Träger umfasst, welcher mindestens einen perforierbaren Deckel (16, 160) aufweist, der die Öffnung jeder Küvette bedeckt, wobei der Deckel eine Ablagefläche (S) für die entnommene Probe aufweist,
- eine dritte Stufe, welche eine Probenahmevorrichtung aufweist, die einen oder mehrere Ausstecher (12, 120) umfasst, die in Bezug auf den Körper beweglich sind,
- wobei jeder Ausstecher (12, 120) mit dem Körper in Translation entlang einer Hauptrichtung (A) zwischen mindestens einem Ruhezustand, in welchem sich der Ausstecher (12, 120) senkrecht über der Ablagefläche (S) befindet, und einem betätigten Zustand, in welchem sich der Ausstecher im Inneren der Küvette (13, 130) befindet, zusammenwirkt, und dadurch, dass:
- der Körper eine Anordnung aufweist, die von einem Aufnahmebehälter (10, 100) und einer den Aufnahmebehälter verschließenden Abdeckung (11, 110) gebildet wird, wobei die Anordnung einen inneren Raum (V), in welchem der die Ablagefläche (S) aufweisende Träger angeordnet ist, und einen äußeren Raum begrenzt,
- mechanische Mittel zur translatorischen Betätigung der Ausstecher.

2. Vorrichtung nach Anspruch 1, **dadurch gekennzeichnet, dass** die erste Stufe (15', 150') in Bezug auf die zwei anderen Stufen der Vorrichtung lösbar ist.

3. Vorrichtung nach Anspruch 1, **dadurch gekennzeichnet, dass** sie einen Verbindungskanal (15, 150) aufweist, der mit jeder Küvette (13, 130) verbunden ist.

4. Vorrichtung nach Anspruch 1, **dadurch gekennzeichnet, dass** jeder Ausstecher (12) mit der Abdeckung (11) fest verbunden ist.

5. Vorrichtung nach Anspruch 4, **dadurch gekennzeichnet, dass** die mechanischen Betätigungsmittel einen Gleitführungsmechanismus aufweisen, der zwischen der Abdeckung (11) und dem Aufnahmebehälter (10) angeordnet ist, um die Abdeckung in Bezug auf den Aufnahmebehälter in der Hauptrichtung (A) translatorisch zu führen.

6. Vorrichtung nach Anspruch 1, **dadurch gekennzeichnet, dass** die mechanischen Betätigungsmittel eine bewegliche Anordnung aufweisen, mit der jeder Ausstecher fest verbunden ist, die sich im inneren Raum (V) befindet und die mithilfe eines Greiforgans (180) translatorisch betätigbar ist, wobei ein Gleitführungsmechanismus zwischen der beweglichen Anordnung und dem Körper der Vorrichtung angeordnet ist.

7. Vorrichtung nach einem der Ansprüche 1 bis 6, **dadurch gekennzeichnet, dass** der Deckel (16, 160) eine Kunststoff- oder Metallfolie ist.

8. Vorrichtung nach einem der Ansprüche 1 bis 7, **dadurch gekennzeichnet, dass** sie ein Gitter (17, 170) aufweist, das in dem Aufnahmebehälter am Umfang der Ablagefläche (S) angebracht ist.

9. Vorrichtung nach einem der Ansprüche 1 bis 8, **dadurch gekennzeichnet, dass** jeder Ausstecher von einem hohlen Teil an einem freien Ende eines hohlen rotationszylindrischen Rohres gebildet wird, dessen Achse in der Hauptrichtung ausgerichtet ist.

10. Vorrichtung nach Anspruch 9, **dadurch gekennzeichnet, dass** sie mindestens zwei Ausstecher mit unterschiedlichen Volumina aufweist.

11. Vorrichtung nach einem der Ansprüche 1 bis 10, **dadurch gekennzeichnet, dass** sie zusätzliche Küvetten aufweist, die jeweils dazu bestimmt sind, eine Auflösungs- oder Resuspensionsflüssigkeit (14, 140) aufzunehmen, und nicht einem Ausstecher der Vorrichtung zugeordnet sind.

12. Verwendung der Vorrichtung, wie in einem der Ansprüche 1 bis 11 definiert, um aus ein und derselben entnommenen Probe (P) von menschlichem Stuhl mithilfe jedes Ausstechers (12, 120) eine oder mehrere Proben herzustellen.

13. Verfahren zur Vorbereitung mehrerer kalibrierter Proben aus ein und derselben entnommenen Materialprobe (P), **dadurch gekennzeichnet, dass** es mithilfe der Vorrichtung, wie in einem der Ansprüche 1 bis 12 definiert, durchgeführt wird, und dadurch, dass es die folgenden Schritte umfasst:
- Anbringung der entnommenen Probe (P) auf der Ablagefläche, während sich die Vorrichtung im Ruhezustand befindet,
- Betätigung der Vorrichtung, um jeden Ausstecher (12, 120) in Richtung der Ablagefläche zwischen dem Ruhezustand und dem betätigten Zustand translatorisch anzutreiben,
- Ausschneiden, durch jeden Ausstecher, eines Teils der entnommenen Probe (P), um eine Probe zu erzeugen,
- Durchstoßen, mit jedem Ausstecher (12, 120), des Deckels (16, 160),
- Einführen jedes Ausstechers (12, 120) in eine gesonderte Küvette (13, 130),
- Auflösung oder Resuspendierung der Probe in der Flüssigkeit (14, 140), die in der Küvette (13, 130) vorhanden ist.

## Claims

1. Device for preparing one or more calibrated solid specimens for analysis from the one same material sample (P), **characterized in that** it comprises a body and a structure with three superposed tiers, said structure comprising:
- a first tier comprising one or more cups (13, 130) which are secured to the body and each intended to receive a dissolution or resuspension liquid (14, 140) and that each have an opening,
- a second tier comprising a support secured to the body and comprising at least one perforable membrane (16, 160) covering the opening of each cup, said membrane having a sample deposition surface (S),
- a third tier which comprises a sampling device comprising one or more core cutters (12, 120) able to move relative to the body,
- each core cutter (12, 120) engaging translationally with said body in a main direction (A), moving between at least a state of rest in which the core cutter (12, 120) is vertically above the deposition surface (S) and an actuated state in which the core cutter is situated inside said cup (13, 130), and **in that**:
- the body comprises an assembly formed of a receptacle (10, 100) and of a lid (11, 110) closing said receptacle, said assembly delimiting an internal space (V) in which the support comprising the deposition surface (S) is placed, and an external space,
- mechanical means for actuating the translational movement of said core cutters.

2. Device according to Claim 1, **characterized in that** the first tier (15', 150') is removable relative to the other two tiers of the device.

3. Device according to Claim 1, **characterized in that** it comprises a connecting channel (15, 150) connected to each cup (13, 130).

4. Device according to Claim 1, **characterized in that** each core cutter (12) is secured to the lid (11).

5. Device according to Claim 4, **characterized in that** said mechanical actuating means comprise a guideway mechanism arranged between said lid (11) and said receptacle (10) to guide the translational movement of said lid relative to the receptacle in said main direction (A).

6. Device according to Claim 1, **characterized in that** the mechanical actuating means comprise a mobile assembly to which each core cutter is secured and which is situated inside the internal space (V) and can be actuated in translational movement by a gripping member (180), a guideway mechanism being arranged between said mobile assembly and the body of the device.

7. Device according to one of Claims 1 to 6, **characterized in that** the membrane (16, 160) is a plastic or metal film.

8. Device according to one of Claims 1 to 7, **characterized in that** it comprises a grating (17, 170) arranged in said receptacle, at the periphery of the deposition surface (S).

9. Device according to one of Claims 1 to 8, **characterized in that** each core cutter is produced from a hollow part at a free end of a hollow tube that is a cylinder of revolution and the axis of which is directed in the main direction.

10. Device according to Claim 9, **characterized in that** it comprises at least two core cutters with distinct volumes.

11. Device according to one of Claims 1 to 10, **characterized in that** it comprises additional cups each intended to receive a dissolution or resuspension liquid (14, 140) and which are not associated with a core cutter of the device.

12. Use of the device as defined in one of Claims 1 to 11 for creating one or more specimens from the one same human-stool sample (P) using each core cutter (12, 120).

13. Method for preparing several calibrated specimens from the one same material sample (P), **characterized in that** it is implemented using the device as defined in one of Claims 1 to 12, and **in that** it comprises the following steps:
- placing the sample (P) on the deposition surface while the device is in the state of rest,
- actuating the device to drive each core cutter (12, 120) translationally towards the deposition surface, between the state of rest and the actuated state,
- cutting out part of the sample (P) using each core cutter in order to create a specimen,
- perforating the membrane (16, 160) using each core cutter (12, 120),
- inserting each core cutter (12, 120) into a distinct cup (13, 130),
- dissolving or resuspending the specimen in the liquid (14, 140) present in the cup (13, 130).
